# EUROPÄISCHE PATENTSCHRIFT

(11) **EP 1 233 797 B1**
(45) Veröffentlichungstag und Bekanntmachung des Hinweises auf die Patenterteilung: **19.07.2006**
(21) Anmeldenummer: 00984957.1
(22) Anmeldetag: 03.11.2000
(51) Int. Cl.: A61M 1/10

(54) **INTRAVASALE BLUTPUMPE**
INTRAVASCULAR BLOOD PUMP
POMPE A SANG INTRAVASCULAIRE

(30) Priorität: 04.12.1999 DE 29921352 U
(43) Veröffentlichungstag der Anmeldung: 28.08.2002
(73) Patentinhaber: Impella CardioSystems GmbH, 52074 Aachen (DE)
(72) Erfinder: SIESS, Thorsten, 52146 Würselen (DE)
(74) Vertreter: Selting, Günther
(86) Internationale Anmeldenummer: PCT/EP2000/010863
(87) Internationale Veröffentlichungsnummer: WO 2001/039817

(56) Entgegenhaltungen:
- EP-A- 0 764 448
- WO-A-97/37696
- WO-A-98/44619
- DE-A- 19 622 335

## Beschreibung

Die Erfindung betrifft eine intravasale Blutpumpe mit einem einen Elektromotor enthaltenden Gehäuse, das am proximalen Ende mit einem Katheter verbunden ist und am distalen Ende eine Pumpe trägt. Eine intravasale Blutpumpe ist eine Blutpumpe, die durch das Gefäßsystem eines Patienten hindurchgeschoben werden kann, um im Herzen des Patienten oder an anderer Stelle des Körpers betrieben zu werden.

Aus WO 98/44619 ist eine intravasale Blutpumpe bekannt, die die genannten Voraussetzungen erfüllt. Die Blutpumpe weist ein Gehäuse mit einem Durchmesser von maximal 7 mm auf, das einen Elektromotor enthält, welcher das Flügelrad einer Pumpe treibt. Der Elektromotor weist einen Stator mit innen angeordneten Wicklungen und außen angeordneten magnetischen Rückschlußblechen auf. Die Rückschlußbleche sind zusammen mit der Statorwicklung in einem aus Kunststoff bestehenden Gehäuse vergossen. Die Rückschlußbleche haben die Wirkung, dass sie den Magnetfluss des umlaufenden Magnetfeldes konzentrieren und unter Vermeidung magnetischer Streuverluste den Wirkungsgrad des Elektromotors vergrößern.

DE 196 22 335 A1 beschreibt einen Ballonkatheter mit einer Pumpe, welche von einem im Verlauf des Katheterschlauchs angeordneten Motor angetrieben sein kann. Ein Führungskanal für einen Führungsdraht verläuft durch den Katheterschlauch und koaxial durch die Pumpe. Auf diese Weise kann der Ballonkatheter zusammen mit der Pumpe über einen zuvor verlegten Führungsdraht geschoben werden, um die Pumpe an der gewünschten Stelle im Patientenkörper zu plazieren.

In US 5 851 174 und EP 0 764 448 A2 ist jeweils eine cardiale Unterstützungseinrichtung beschrieben, die eine intrakardiale Blutpumpe aufweist, welche durch eine Inzisionsstelle in das Herz eingeführt werden kann. Die Blutpumpe hat einen Außendurchmesser von 9 bis 12 mm. Dies erfüllt nicht die Anforderungen an eine intravasale Blutpumpe. Der Motor weist einen in das Gehäuse integrierten Stator mit einer Statorwicklung auf und einen magnetischen Rotor. Der Rotor ist mit Flügeln versehen, welche in den Ringraum zwischen Stator und Rotor hineinragen. Daher muß der Ringraum eine große Breite haben, was den magnetischen Luftspalt zwischen Rotor und Stator vergrößert und die Leistungsfähigkeit des Motors bzw. den Wirkungsgrad beeinträchtigt.

Aus EP 0 764 448 A2 ist eine cardiale Blutpumpe bekannt, die einen integrierten Elektromotor aufweist. Der Motor hat einen breiten Luftspalt in welchem Pumpenflügel angeordnet sind. Der Luftspalt ist von Blut durchflossen. Der Stator des Motors ist eisenlos ausgebildet. Er besteht lediglich aus Drahtwicklungen, die zwischen zwei Hülsen abdichtend eingeschlossen sind. Die eisenlose Ausbildung des Stators verfolgt den Zweck, einen breiteren Luftspalt zu schaffen, um die Blutströmung zu verbessern und Blutschädigungen zu vermeiden.

Eine intravasale Blutpumpe, bei der die Antriebseinheit mit dem Pumpenteil vereinigt ist, muss einen möglichst geringen maximalen Außendurchmesser und eine kleine rigide Länge aufweisen. Bisher ist es nicht gelungen, eine Blutpumpe mit der erforderlichen Leistung zu konstruieren, deren Außendurchmesser kleiner ist als 5 mm. Bei einer intravasalen Blutpumpe ist eine Förderleistung von 1,5 l pro Minute bei physiologischen Drücken (60 bis 80 mm Hg) erforderlich.

Der Erfindung liegt die Aufgabe zugrunde, eine intravasale Blutpumpe zu schaffen, die einen noch stärkere Miniaturisierung ermöglicht, um leicht und sicher durch das Gefäßsystem vorgeschoben zu werden.

Die Lösung dieser Aufgabe erfolgt erfindungsgemäß mit den im Patentanspruch 1 angegebenen Merkmalen. Die Besonderheit der erfindungsgemäßen Blutpumpe besteht darin, dass der Stator des Elektromotors ohne magnetische Rückschlußvorrichtung eisenlos ausgeführt ist und dass die Statorwicklung tragender Bestandteil des Motorgehäuses ist. Erfindungsgemäß bildet die in eine Kunstharzmatrix eingebettete Statorwicklung den tragenden Teil des Motorgehäuses. Der in der Statorwicklung fließende Strom erzeugt ein umlaufendes Magnetfeld, welchem die an dem Rotor befestigte Magnetvorrichtung folgt. Der Verzicht auf Polschuhe und auf eine eisenbehaftete Rückschlußvorrichtung hat zur Folge, dass das in der Statorwicklung erzeugte Magnetfeld außerhalb der Statorwicklung nicht gebündelt wird und dass somit Streuverluste nach außen entstehen. Gezielt durchgeführte Untersuchungen haben ergeben, dass das mit hoher Drehzahl umlaufende Magnetfeld keinen schädlichen Einfluss auf den Herzrhythmus des Patienten hat. Die Drehzahl des Magnetfeldes und somit auch diejenige des Elektromotors liegt in der Größenordnung von 50.000 U/min und beträgt in jedem Fall mehr als 30.000 U/min. Bei dieser hohen Drehzahl treten durch das rotierende Magnetfeld keine schädigenden Wirkungen im Patientenkörper auf. Bei der erfindungsgemäßen Blutpumpe wird der Platz für die Rückschlußvorrichtung eingespart. Dadurch kann der Durchmesser des Rotors vergrößert werden, was eine Erhöhung des Drehmoments zur Folge hat. Außerdem werden Ummagnetisierungsverluste, die in einer aus Eisen bestehenden Rückschlußvorrichtung auftreten, vermieden. Dadurch werden bei den genannten hohen Drehzahlen die erhöhten Streuverluste mehr als ausgeglichen. Schließlich wird auch das Gewicht des Motors verringert. Die eisenlose Ausbildung des Stators erlaubt es auch, für die Statorwicklung eine größere Drahtstärke zu verwenden, so dass die Stromstärke vergrößert werden kann bei gleichzeitig geringen Ohmschen Verlusten in der Statorwicklung.

Bei der erfindungsgemäßen Blutpumpe kann das den Elektromotor enthaltende Gehäuse auf einen Außendurchmesser von etwa 4 mm reduziert werden, was einem Katheter von 12 F (F = French) entspricht. Die rigide axiale Länge der gesamten Pumpe, also des Motorgehäuses und des Pumpenteils, kann auf ≤ 20 mm reduziert werden. Somit kann die intravasale Blutpumpe durch das Blutgefäßsystem des Patienten vorgeschoben und an der gewünschten Stelle plaziert werden. Die Abmessungen sind auch dazu geeignet, die Blutpumpe behinderungsfrei und gesteuert durch den Aortenbogen zu manövrieren.

Die Statorwicklung nimmt mindestens 70 % - vorzugsweise mindestens 80 % - der Wandstärke des Gehäuses ein. Die Statorwicklung bildet zusammen mit der Kunstharzmatrix das tragende Strukturteil der Gehäusewand. Generell reicht es aus, wenn die Statorwicklung zusammen mit der Kunstharzmatrix das Gehäuse bildet. Es ist jedoch zweckmäßig, an der Innenseite der Gehäusewand eine schützende Isolationsschicht anzuordnen, die auch als Reibschutz für die Wicklung dient, um zu verhindern, dass der Rotor die Wicklung bei mechanischem Kontakt beschädigt. Ferner kann an der Außenseite des Gehäuses eine zusätzliche schützende Folie angebracht sein, die gute wärmeleitende Eigenschaften haben muss, um die in der Statorwicklung erzeugte Wärme an das außen entlangströmende Blut abzugeben.

Um die Blutpumpe zusammen mit dem zugehörigen Katheter nach Art der Seldinger-Technik verlegen zu können, muss die Blutpumpe einen Führungskanal aufweisen, durch den ein Führungsdraht geschoben werden kann. Die Blutpumpe kann dann entlang dem Führungsdraht im Gefäßsystem des Patienten vorgeschoben werden. Die erfindungsgemäße Blutpumpe ist zweckmäßigerweise mit einem solchen Führungskanal ausgestattet, der durch die Welle des Rotors und durch die Achse des Flügelrades der Pumpe hindurchgeht. Dabei können entsprechende Dichtungen vorgesehen sein, die verhindern, dass das den Führungskanal eindringende Blut in den proximal vom Gehäuse angeordneten Katheter und in die Mechanik der Pumpe gelangt.

Im folgenden werden unter Bezugnahme auf die Zeichnungen Ausführungsbeispiele der Erfindung näher erläutert.

Es zeigen:
- Fig. 1: einen Längsschnitt durch eine erste Ausführungsform der intravalen Blutpumpe mit voller Welle,
- Fig. 2: einen Querschnitt durch die Gehäusewand entlang der Linie II-II von Fig. 1,
- Fig. 3: einen Längsschnitt durch eine zweite Ausführungsform der Blutpumpe mit Führungskanal für einen Führungsdraht,
- Fig. 4: eine Modifikation der Pumpe nach Fig. 3 mit einer geänderten Dichtungsanordnung zur Abdichtung des Führungskanals, und
- Fig. 5: eine Darstellung der Dichtungsanordnung von Fig. 4 mit hindurchgestecktem Führungsdraht.

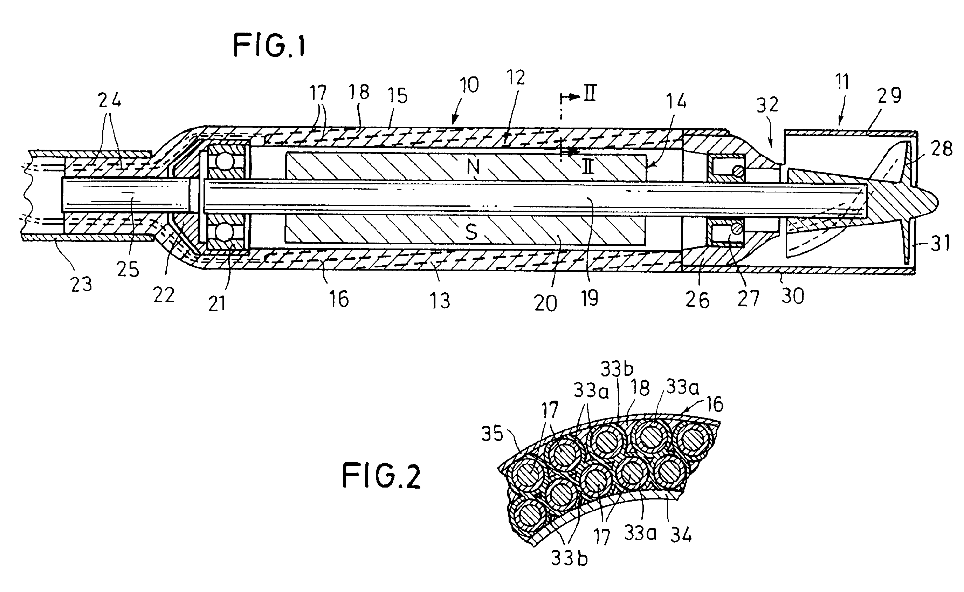

Die in Fig. 1 dargestellte Blutpumpe besteht aus einem Motorteil 10 und einem Pumpenteil 11. Der Motorteil 10 weist einen Elektromotor 12 auf, der aus einem Stator 13 und einem Rotor 14 besteht. Der Stator 13 bildet zugleich die Umfangswand des Gehäuses 15, d.h. es ist kein zusätzliches den Stator umgebendes Gehäuse vorhanden. Die den Stator 13 bildende Gehäusewand 16 enthält eine Statorwicklung 17 aus Drähten, die in eine Kunstharzmatrix 18 eingebettet sind. Die Wicklung 17 ist eine sogenannte Basket-Wicklung oder Korbwicklung mit schräg in Längsrichtung des Gehäuses 15 verlaufenden Drähten. Die Statorwicklung 17 enthält mehrere umfangsmäßig verteilt angeordnete und im Stern oder Dreieck verschaltete Spulen, die nacheinander erregt werden und dadurch ein umlaufendes Magnetfeld erzeugen. Die sensorlose Kommutierung dieser Spulen erfolgt durch ein Steuergerät, das extrakorporal angeordnet ist und das mit dem Motor über eine mehradrige Drahtleitung 24 verbunden ist. Die Wicklung 17 wird in der Weise erregt, dass das umlaufende Magnetfeld mit einer Drehzahl von 50.000 U/min rotiert.

Der Rotor 14 weist einen auf der Welle 19 befestigten Permanentmagneten 20 auf, der auf der einen Seite seines Umfangs den Nordpol N und auf der anderen Seite den Südpol S aufweist.

Die Welle 19 ist an dem proximalen Ende in einem Kugellager 21 gelagert. Das Kugellager ist in einer Kappe 22 gehalten, die das proximale Ende des Gehäuses 15 abschließt. An das proximale Ende des Gehäuses 15 schließt sich der Katheter 23 an, der aus einem flexiblen Schlauch besteht. Durch die Wand des Katheters 23 verlaufen die Speiseleitungen 24 für die Statorwicklung 17. Die Gehäusekappe 22 ist bei dem vorliegenden Ausführungsbeispiel mit einem in den Katheter 23 hineinragenden Ansatz 25 verbunden, um eine hohe mechanische Stabilität zu erlangen.

Das distale Ende des Gehäuses 15 ist mit einem Übergangsstück 26 abgeschlossen, das eine zugleich als Wellenlager dienende Dichtung 27 enthält. Die Welle 19 erstreckt sich durch diese Dichtung hindurch in den Pumpenteil 11 hinein, wo das Flügelrad 28 an dem distalen Wellenende befestigt ist. Das Flügelrad 28 rotiert in dem zylindrischen Pumpengehäuse 29, das mit dem Gehäuse 15 über mindestens einen längs laufenden Steg 30 verbunden ist. Das Pumpenteil 11 saugt Blut über die axiale Einlassöffnung 31 an und fördert in axialer Richtung, wobei das Blut aus den Öffnungen 32 seitlich austritt und dann axial an dem Gehäuse 15 entlangströmt, wodurch die Stromwärme von dem Gehäuse 15 abgeführt wird. Die Pumpe kann aber auch in entgegengesetzter Richtung betrieben werden.

Bei dem Ausführungsbeispiel von Fig. 1 ist die Welle 19 eine Vollwelle. Die rigide Länge der gesamten Blutpumpe, über den Motorteil und den Pumpenteil 11 beträgt etwa 20 mm. Der größte Außendurchmesser beträgt 4 mm.

Aus Fig. 2 sind die in die Kunstharzmatrix 18 der Gehäusewand 16 hohlraumfrei eingebetteten Drähte der Wicklung 17 erkennbar. Diese Drähte sind mit einem Isoliermantel 33a umgeben. Die Isoliermäntel sind ihrerseits mit einem Back-Lack 33b beschichtet. Nach dem Herstellen der Statorwicklung 17 werden die Drähte unter Wärmeeinwirkung miteinander verklebt, wobei die Back-Lack-Schichten benachbarter Drähte miteinander verschmelzen, so dass eine fixierte Drahtstruktur entsteht. Diese wird anschließend in einer Spritzgußform mit dem Kunstharz 18 ausgespritzt, bei dem es sich um ein Epoxydharz handelt. Dieser Verbund aus Wicklung und Epoxydharz ist mechanisch sehr stabil, so dass der Back-Lack 33b ausschließlich als Vorfixierung der Wicklungsdrähte dient, um die Handhabung der Spule während des Fertigungsprozesses zu erleichtern. Entsprechend dünnwandig kann der Back-Lack ausgeführt werden, so dass ein optimales Verhältnis zwischen Kupfer, Back-Lack, Isolation und Expoxydharz erreicht werden kann. Denn nur der Kupferanteil der Wicklung bestimmt die Leistungsfähigkeit des Motors.

Nachdem auf diese Weise das Gehäuse 15 hergestellt wurde, kann an dem Gehäuse der Katheter 23 befestigt werden. Das Lager 21 hat einen Außendurchmesser der kleiner ist als der Innendurchmesser des Gehäuses 15, so dass es vom distalen Ende her in das Gehäuse eingeschoben werden kann.

An der Innenseite der Gehäusewand 16 befindet sich eine dünne Isolationsschicht 34 aus einer Kaptonfolie (Polyimid) mit einer Stärke von 12 bis 25 µm. Diese Isolationsschicht dient als Isolation und Reibschutz für die Drähte zur Verhinderung von Beschädigungen durch den Rotor und als Gleitschicht.

An der Außenseite der Gehäusewand 16 befindet sich eine Mantelfolie 35 aus einem gut wärmeleitenden Material, vorzugsweise aus einem Metall, wie Titan. Anstelle der Mantelfolie kann auch die Kunstharzmatrix 18 nach außen erweitert sein.

Die Kunstharzmatrix 18 besteht aus einem Duromer mit einem Gewichtsanteil von mindestens 50 Gewichtsprozent Al₂O₃, um die Wärmeabfuhr nach außen zu erhöhen. Die Stärke der Gehäusewand 16 beträgt einschließlich der Schichten 34 und 35 etwa 0,3 mm. Die Statorwicklung 17 nimmt mindestens 70 %, vorzugsweise mindestens 80% der Wandstärke der Gehäusewand 16 ein.

Der Motor ist zahnlos, d.h. er weist keine umfangsmäßig verteilten Polschuhe auf, welche Wicklungen tragen. Die Statorwicklung ist vielmehr in die Wand des rohrförmigen Gehäuses 15 integriert und trägt dort maßgeblich zur mechanischen Stabilität der Gehäusewand 16 bei.

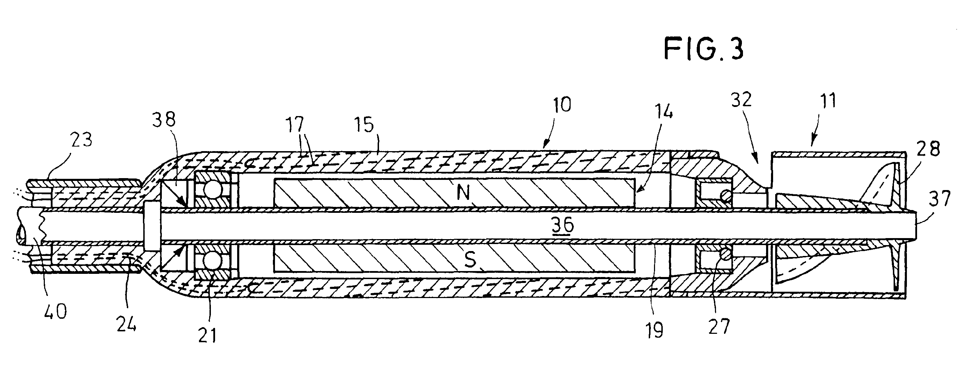

Das Ausführungsbeispiel von Fig. 3 unterscheidet sich von dem ersten Ausführungsbeispiel dadurch, dass die Welle 19 als Hohlwelle ausgebildet ist und einen Führungskanal 36 enthält, der sich über die gesamte Länge der Welle erstreckt. Der Führungskanal 36 steht mit dem Lumen des Katheters 23 über einen weiteren Schlauch 40 in Verbindung.

An dem distalen Ende der Welle 19 befindet sich eine Öffnung 37. Durch diese Öffnung kann Blut in den Führungskanal 36 eintreten. Um zu verhindern, dass dieses Blut in das Innere des Elektromotors eindringt, ist proximal von dem Lager 21 eine Dichtung 38 vorgesehen.

Die Blutpumpe von Fig. 3 kann über einen (nicht dargestellten) Führungsdraht geschoben werden, der durch den Katheter 23 und den Führungskanal 36 hindurch verläuft und zuvor im Patientenkörper plaziert wird. Im übrigen gelten für das Ausführungsbeispiel von Fig. 3 die gleichen strukturellen Merkmale und Wirkungen wie für das erste Ausführungsbeispiel.

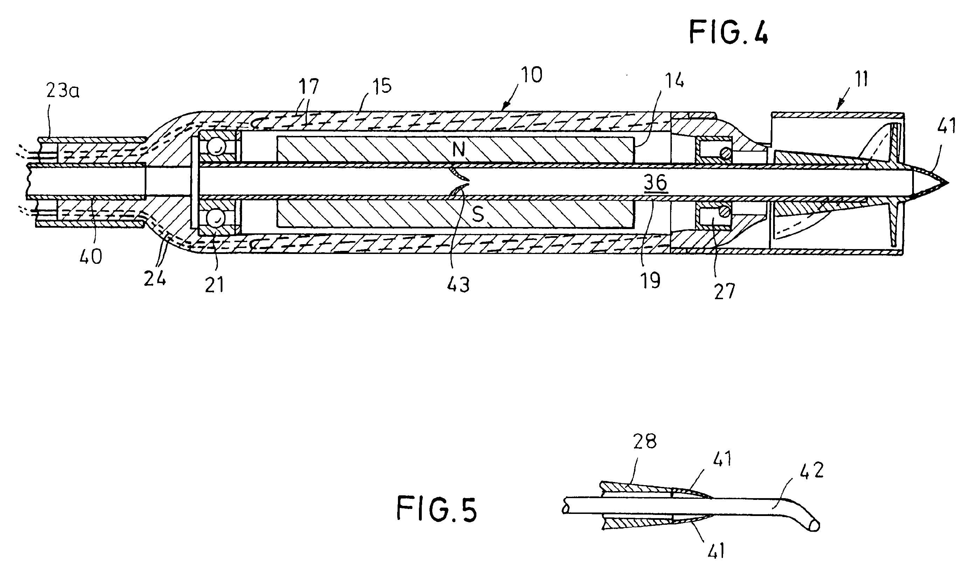

Das Ausführungsbeispiel der Fign. 4 und 5 weist ebenfalls eine Hohlwelle 19 auf, die sich durch den Motorteil 10 und den Pumpenteil 11 erstreckt und einen Führungskanal 36 für einen Führungsdraht bildet. An das proximale Ende der Welle 19 schließt sich ein in dem Katheter 23 verlaufender Schlauch 40 an, der mit dem Gehäuse 15 verbunden ist und nicht mit der Welle 19 dreht.

Am distalen Ende des Führungskanals 36 ist eine Dichtung 41 vorgesehen, die den Führungskanal 36 abdichtet. Bei der Dichtung 41 handelt es sich um eine selbstschließende Lippendichtung. Diese weist eine Öffnung für den Durchtritt des Führungsdrahtes 42 auf. In Fig. 5 ist dargestellt, wie der Führungsdraht 42 die Öffnung der Dichtung 41 aufgestoßen hat. Der Führungsdraht 42 kann also in der Dichtung 41 axial verschoben werden. Wird er nach proximal herausgezogen, dann schließt die Dichtung 41 selbsttätig.

Eine entsprechende selbstschließende Dichtung 43 ist im Innern der Welle 19 vorgesehen. Diese Dichtung 43 dient als zusätzliche Sicherung. Das Lumen des Führungskanals 36 hat einen Durchmesser von 0,6 mm, so dass ein Führungsdraht entsprechender Stärke hindurchgeführt werden kann.

## Patentansprüche

1. Intravasale Blutpumpe mit einem einen Elektromotor (12) enthaltenden langgestreckten Gehäuse (15), das am proximalen Ende mit einem Katheter (23) verbunden ist und am distalen Ende eine Pumpe (11) trägt, wobei der Elektromotor (12) eine in eine Kunstharzmatrix (18) eingebettete Statorwicklung (17) und einen drehbar gelagerten magnetischen flügellosen Rotor (14) aufweist,
**dadurch gekennzeichnet,**
**dass** die Statorwicklung (17) tragender Bestandteil des Gehäuses (15) ist, welches ohne magnetische Rückschlußvorrichtung eisenlos ausgeführt ist.

2. Blutpumpe nach Anspruch 1, **dadurch gekennzeichnet, dass** die Statorwicklung (17) mindestens 70 % - vorzugsweise mindestens 80 % - der Wandstärke des Gehäuses (15) einnimmt.

3. Blutpumpe nach Anspruch 1 oder 2, **dadurch gekennzeichnet, dass** die Wandstärke des Gehäuses (15) kleiner ist als 0,5 mm.

4. Blutpumpe nach einem der Ansprüche 1-3, **dadurch gekennzeichnet, dass** der Elektromotor (12) eine hohle Welle (19) aufweist und durch die Achse des Flügelrades (28) der Pumpe (11) ein Führungskanal (36) für einen Führungsdraht verläuft.

5. Blutpumpe nach einem der Ansprüche 1-4, **dadurch gekennzeichnet, dass** am distalen Ende und am proximalen Ende des Gehäuses (15) Dichtungen (27,38) zwischen Gehäuse (15) und Welle (19) angeordnet sind.

6. Blutpumpe nach Anspruch 4, **dadurch gekennzeichnet, dass** am distalen Ende des Gehäuses (15) eine Dichtung (27) zwischen Gehäuse und Welle (19) angeordnet ist, und dass mindestens eine von dem Führungsdraht (42) passierbare weitere Dichtung (41,43) im Verlaufe des Führungskanals (36) vorgesehen ist.

7. Blutpumpe nach einem der Ansprüche 1-6, **dadurch gekennzeichnet, dass** an der Innenseite des Gehäuses (15) eine die Statorwicklung (17) schützende Isolationsschicht (34) angeordnet ist.

8. Blutpumpe nach einem der Ansprüche 1-7, **dadurch gekennzeichnet, dass** an der Außenseite des Gehäuses (15) eine die Statorwicklung (17) schützende Mantelschicht (35) zur elektrischen Isolation und zur Wärmeabfuhr angeordnet ist.

9. Blutpumpe nach einem der Ansprüche 1-8, **dadurch gekennzeichnet, dass** die Drähte der Statorwicklung (17) einen Isoliermantel (33a) aufweisen, der von einer Back-Lack-Schicht (33b) umgeben ist.

10. Blutpumpe nach einem der Ansprüche 1-9, **dadurch gekennzeichnet, dass** das proximale Ende der Welle (19) in einem Lager (21) gelagert ist, dessen Außendurchmesser kleiner ist als der Innendurchmesser des Stators (13).

## Claims

1. An intravascular blood pump comprising an elongate housing (15) accommodating an electric motor (12), the proximal end of the housing (15) being connected to a catheter (23) and the distal end thereof carrying a pump (11), the electric motor (12) comprising a stator winding (17) embedded in matrix (18) made from synthetic resin, and a bladeless magnetic rotor (14) supported for rotation,
**characterized in**
**that** the stator winding (17) forms a load-bearing component of the housing (15), the housing being made ironless without any magnetic reflux device.

2. The blood pump according to claim 1, **characterized in that** the stator winding (17) occupies at least 70% - preferably at least 80% - of the wall thickness of housing (15).

3. The blood pump according to claim 1 or 2, **characterized in that** the wall thickness of the housing (15) is smaller than 0.5 mm.

4. The blood pump according to any one of claims 1-3, **characterized in that** the electric motor (12) comprises a hollow shaft (19) and that the axis of the impeller (28) of the pump (11) is formed with a guide channel (36) for a guide wire.

5. The blood pump according to any one of claims 1-4, **characterized in that** sealings (27,38) are arranged at the distal end and the proximal end of the housing (15) between the housing (15) and the shaft (19).

6. The blood pump according to claim 4, **characterized in that** a sealing (27) is arranged at the distal end of the housing (15) between the housing and the shaft (19), and that at least one further sealing (41,43) is arranged in the course of the guide channel (36) and allowing passage of the guide wire (42) therethrough.

7. The blood pump according to any one of claims 1-6, **characterized in that** an insulating layer (34) protecting the stator winding (17) is arranged on the inner side of the housing (15).

8. The blood pump according to any one of claims 1-7, **characterized in that** a cover layer (35) protecting the stator winding (17) and provided for electric insulation and heat dissipation is arranged on the outer side of the housing (15).

9. The blood pump according to any one of claims 1-8, **characterized in that** the wires of the stator winding (17) are provided with an insulating sheathing (33a) surrounded by a stoving paint layer (33b).

10. The blood pump according to any one of claims 1-9, **characterized in that** the proximal end of the shaft (19) is supported in a bearing (21) having an outer diameter smaller than the inner diameter of the stator (13).

## Revendications

1. Pompe sanguine intravasculaire, comportant un boîtier (15) allongé, qui contient un moteur électrique (12) et dont l'extrémité proximale est reliée à un cathéter (23) et dont l'extrémité distale porte une pompe (11), le moteur électrique (12) comportant un enroulement de stator (17) enrobé dans une matrice de résine synthétique (18) et un rotor (14) magnétique sans ailette, monté rotatif,
**caractérisée en ce que** l'enroulement de stator (17) est une partie intégrante portante du boîtier (15), qui est réalisé exempt de fer et sans dispositif de blindage magnétique.

2. Pompe sanguine selon la revendication 1, **caractérisée en ce que** l'enroulement de stator (17) occupe au moins 70 % - de préférence au moins 80 % - de l'épaisseur de la paroi du boîtier (15).

3. Pompe sanguine selon la revendication 1 ou 2, **caractérisée en ce que** l'épaisseur de paroi du boîtier (15) est inférieure à 0,5 mm.

4. Pompe sanguine selon l'une quelconque des revendications 1 à 3, **caractérisée en ce que** le moteur électrique (12) comporte un arbre creux (19), et **en ce qu'**un canal de guidage (36) pour un fil de guidage passe à travers l'axe de la turbine (28) de la pompe (11).

5. Pompe sanguine selon l'une quelconque des revendications 1 à 4, **caractérisée en ce que** des garnitures d'étanchéité (27, 38) entre le boîtier (15) et l'arbre (19) sont agencées au niveau de l'extrémité distale et de l'extrémité proximale du boîtier (15).

6. Pompe sanguine selon la revendication 4, **caractérisée en ce qu'**une garniture d'étanchéité (27) est agencée sur l'extrémité distale du boîtier (15) entre le boîtier et l'arbre (19), et **en ce qu'**il est prévu dans le tracé du canal du guidage (36) au moins une garniture d'étanchéité supplémentaire (41, 43) à travers laquelle peut passer le fil de guidage (42).

7. Pompe sanguine selon l'une quelconque des revendications 1 à 6, **caractérisée en ce qu'**une couche isolante (34), protégeant l'enroulement de stator (17), est agencée sur la face intérieure du boîtier (15).

8. Pompe sanguine selon l'une quelconque des revendications 1 à 7, **caractérisée en ce qu'**une couche enveloppante (35), protégeant l'enroulement de stator (17) et destinée à l'isolation électrique et à l'évacuation de la chaleur, est agencée sur la face extérieure du boîtier (15).

9. Pompe sanguine selon l'une quelconque des revendications 1 à 8, **caractérisée en ce que** les fils de l'enroulement du stator (17) comportent une gaine isolante (33a), qui est entourée par une couche de « back-lack » (33b).

10. Pompe sanguine selon l'une quelconque des revendications 1 à 9, **caractérisée en ce que** l'extrémité proximale de l'arbre (19) est montée dans un palier (21) dont le diamètre extérieur est inférieur au diamètre intérieur du stator (13).
